# EUROPÄISCHE PATENTSCHRIFT

(11) **EP 0 625 341 B1**
(45) Veröffentlichungstag und Bekanntmachung des Hinweises auf die Patenterteilung: **01.12.1999**
(21) Anmeldenummer: 93902594.6
(22) Anmeldetag: 18.12.1992
(51) Int. Cl.: A61F 5/01, A63B 21/055

(54) **VORRICHTUNG ZUR BEHANDLUNG VON PATIENTEN MIT PROBLEMEN DER HALTUNG SOWIE DER MOTORISCHEN AKTIVITÄTEN**
DEVICE FOR TREATING PATIENTS WITH DISTURBANCES OF POSE AND MOTOR ACTIVITY
DISPOSITIF DE TRAITEMENT DE PATIENTS PRESENTANT DES TROUBLES D'ATTITUDE ET D'ACTIVITE MOTRICE

(30) Priorität: 31.01.1992 RU 5025647
(43) Veröffentlichungstag der Anmeldung: 23.11.1994
(73) Patentinhaber: AKTSIONERNOE OBSCHESTVO ZAKRYTOGO TIPA "AJURVEDA", Moscow, 117593 (RU)
(72) Erfinder: AFANASENKO, Nikolai Ivanovich, Moscow, 111587 (RU); BARER, Arnold Semenovich, Moscow, 129301 (RU); GRIGORIEV, Anatoly Ivanovich, Moscow, 125047 (RU); KOZLOVSKAYA, Inesa Benediktovna, Moscow, 103006 (RU); SAVINOV, Albert Pavlovich ul. Pionerskaya, 19-8, pos. Tomilino, 140070 (RU); SEVERIN, Gai Iliich, Moscow, 117296 (RU); SEMENOVA, Xenia Alexandrovna, Moscow, 117330 (RU); SINIGIN, Viktor Mikhailovich, Moskovskaya obl. pos. Tomilino, 140070 (RU); SOKOLOVSKY, Igor Antonovich ul. Gogolya, 48-66, pos. Tomilino, 140070 (RU); TIKHOMIROV, Evgeny Petrovich, Moscow, 115470 (RU)
(74) Vertreter: Hano, Christian, Dipl.-Ing.
(86) Internationale Anmeldenummer: RU9200247
(87) Internationale Veröffentlichungsnummer: WO9315706

(56) Entgegenhaltungen:
- EP-A- 0 066 028
- FR-A- 480 383
- FR-A- 2 120 500
- FR-A- 2 298 314
- FR-A- 2 390 152
- SU-A- 1 528 483
- SU-A- 1 556 675
- US-A- 2 772 674

## Beschreibung

Die Erfindung betrifft eine Vorrichtung nach dem Oberbegriff des Anspruchs 1.

Eine solche Vorrichtung ist aus der SU-A-1 528 483 bekannt. Die Vorrichtung hat einen Brust-, einen Beckenhalter, Fußhalter und Fixierungselemente zur Verbindung der Halter miteinander. Die Fixierungselemente sind als Teleskopstützen ausgebildet, die die Fußhalter und den Beckenhalter sowie eine Stange verbinden, deren eines Ende mit dem Beckenhalter starr verbunden ist. An der Stange ist eine Rolle hin- und herbewegbar gelagert, die mit dem Brusthalter in starrer Verbindung steht. Mit dem Beckenhalter sind zwei Kragstücke fest gekoppelt, deren freie Enden mit Federn verbunden sind, weiche an den Teleskopstützen verschiebbar befestigt sind.

Eine solche Vorrichtung ist ebenfalls aus der US-A-2 772 674 bekannt.

Bei einer vertikalen Körperstellung sorgt die Rolle für einen leichten reklinierenden Effekt an der ganzen Wirbelsäule, wobei der Brusthalter die Unterstützung des oberen Teils des Rumpfes gewährleistet. Beim Rumpfbeugen rollt die Rolle je nach dem Neigungswinkel auf der Oberfläche der Stange ab, indem sie eine optimale Lage einnimmt, während die Federn eine Kraft auf die Stange übertragen. Auf diese Weise erfolgt ein Gewichtsausgleich des gebeugten Rumpfteils und eine Entlastung des Muskelapparats und der Wirbelsäule.

Ein Nachteil dieser Vorrichtung besteht darin, daß sie nur zur Behandlung der Wirbelsäule durch deren Entlastung bestimmt ist. Darüber hinaus kann die Benutzung dieser Vorrichtung zur beschränkten Beweglichkeit des Patienten mit nachfolgender Muskelatrophie und Verringerung der Aktivität der Antigravitations-Muskulatur führen. Der Behandlungsprozeß ist mit dieser Vorrichtung langwierig.

Bekannt ist ferner eine Vorrichtung zur Behandlung von Patienten mit Problemen der Haltung sowie der motorischen Aktivität (FR-A-2 120 500), die in Form eines Overalls ausgeführt ist, in den zur Gewährleistung der Steifigkeit flexible aufblasbare Röhrchen eingesetzt sind.

Ein Nachteil dieser Vorrichtung ist, daß sie zur Beibehaltung einer bestimmten Stellung des Körpers des Patienten bestimmt ist, weshalb das Einsatzgebiet dieser Vorrichtung äußerst beschränkt ist. Überdies ist bel der Verwendung dieser Vorrichtung die Frage eines Trainings des Muskelsystems des Patienten nicht gelost, was zu einer tiefgreifenden Störung von dessen Funktion führen kann.

Eine andere bekannte Vorrichtung zur Behandlung von Patienten mit Problemen der Haltung sowie der motorischen Aktivität (FR-A-2 252 836) weist zwei Schaufeln, die zwischen den Oberschenkeln des Patienten angeordnet sind und die jeweils am entsprechenden Oberschenkel befestigt werden, und ein mit den Schaufein verbundenes mechanisches System auf.

Ein Nachteil dieser Vorrichtung ist, daß sie lediglich eine unrichtige Stellung der Oberschenkel, Kniegelenke und Füße korrigiert. Außerdem ist diese Vorrichtung sperrig, wehalb deren Verwendung für eine Behandlung recht problematisch ist.

Aus der FR-A-480 383 ist eine funktionelle Prothese gegen den Verlust der Bewegungsfähigkeit bekannt. Diese Konstruktion stellt ein System von Federn dar, die in Röhren eingesetzt sind und Anschnallgurte verbinden. Dieses System arbeitet auf der Basis des natürlichen Bestrebens der Federn, sich geradezubiegen. Durch Lageänderung der Befestigungspunkte der Federn kann man die Stellung der Extremitäten verändern. Dies ist jedoch nur in Richtung der Streckung der Extremitäten möglich, d.h. die genannten Federn strecken lediglich die Extremitäten. Außerdem enthält das aus der FR-A-480 383 bekannte System Befestigungselemente (Halter), die aus einer Oberarmmuffe, einem Brust- und Bauchgurt, einem unter dem Arm befindlichen Bügel und einem Schädelgurt bestehen. Per Verwendungszweck der besagten Prothese ist, die fehlende Bewegung bei neurologischen Erkrankungen zu reproduzieren.

Aus der US-A-2 772 674 ist eine orthopädische Torsions-Beinprothese bekannt. Diese Beinprothese enthält drei Umschlingungsriemen in der Taillengegend und Verbindungselemente, die in Längsrichtung (entlang dem Bein) nur auf der Außenseite (Flanke) der beiden Beine angeordnet sind. Ein steifes Verbindungselement, das mittels entsprechender Befestigungseinrichtungen am Schuh angebracht ist, führt vom Schuh bis zu einem auf dem Unterschenkel befindlichen Riemen. Ein weiteres steifes Verbindungselement verläuft vom Leibriemen bis zur Höhe der Frontalachse des Hüftgelenks. Des weiteren sind zwei elastische Verbindungen vorgesehen. Die eine elastische Verbindung führt vom Anschlußpunkt an das eine steife Verbindungselement in Höhe der Hüftgelenkachse bis zum Umschlingungsriemen auf dem Oberschenkel. Die zweite elastische Verbindung führt von dem Riemen auf dem Oberschenkel bis zum Umschlingungsriemen auf dem Unterschenkel. Der funktionelle Bestimmungszweck der aus der US-A-2 772 674 bekannten orthopädischen Torsions-Beinprothese besteht darin, dem Bein eine stabile (gestreckte) Stellung unter Aufrechterhaltung der Funktion der Beugung in dem Knie- und dem Hüftgelenk zu geben. Sie ist in der orthopädischen Praxis anwendbar.

Die FR-A-2 298 314 offenbart eine orthopädische Vorrichtung, die paralysierten Kranken das Stehen ermöglicht. Diese Einrichtung ist in Form eines Overalls ausgebildet, in den zahlreiche Teile in Form von Bändern aus nichtdehnbaren Geweben eingeführt sind, von denen jedes die Zone des Beckens, der Ober- und Unterschenkel straff umspannt. Ferner sind längs der Beine angeordnete aufblasbare Elemente und steife Speichen vorgesehen, die in spezielle Kanäle eingesteckt werden.

Die aus der FR-A-2 298 314 bekannte Vorrichtung ist ausschließlich dazu bestimmt, der vertikalen Stellung des paralysierten Kranken bei wackligen Gelenken Standfestigkeit zu geben. Deshalb ist das Einsatzgebiet dieser Vorrichtung beschränkt. Ein Nachteil der genannten Vorrichtung besteht außerdem darin, daß Strukturelemente für den oberen Rumpfteil und die Arme vollständig fehlen, was es nicht gestattet, auf das Muskelsystem des Schultergürtels einzuwirken. Das kann zu einer tiefgreifenden Störung der Funktion von Muskeln führen. Die aus der FR-A-2 298 314 bekannte Vorrichtung kann nicht unter dynamischen Bedingungen, beispielsweise beim Gehen, verwendet werden.

Aus der FR-A-2 390 152 ist eine orthopädische Vorrichtung bekannt, die paralysierten Kranken das Stehen ermöglicht. Die Vorrichtung ist als Overall ausgebildet, in den zahlreiche Teile aus nichtdehnbaren Geweben in Form von Bändern mit Florverschluß, aufblasbare Kammern, die unter Druck dem Overall Steifigkeit verleihen, und steife Speichen, die in spezielle Kanäle eingesteckt werden und sich in die Gegend des Knie- und des Hüftgelenkes erstrecken, eingeführt sind. Allerdings ist auch bei der aus der FR-A-2 390 152 bekannten Vorrichtung die Frage des Trainings und des Muskelsystems des Schultergürtels nicht gelöst, was zu einer tiefgreifenden Störung der Funktion dieses speziellen Muskelsystems führen kann. Die aus der FR-A-2 390 152 bekannte Vorrichtung ist nur dazu bestimmt, der vertikalen Stellung des paralysierten Kranken bel wackligen Knie- und Hüftgelenken Standfestigkeit zu verleihen. Sie kann unter dynamischen Bedingungen, z.B. beim Gehen, nicht verwendet werden.

Die EP-A-0 066 028 offenbart eine Vorrichtung zur Gewährleistung der vertikalen Stellung und des Gangs. Diese Vorrichtung ist als Overall ausgebildet, der ein Korsett, Kniestücke, Schuhe und zwischen ihnen befindliche elastische Metallplatten enthält, die auf den äußeren Seitenflächen der Oberschenkel und auf den inneren Seitenflächen der Unterschenkel angeordnet sind. Nachteilig ist, daß diese Vorrichtung mit dem großen Korsett einen erheblichen Teil der Körperoberfläche abdeckt, wodurch beim Patienten Wärmeaustauschprobleme in dem entsprechenden Körperabschnitt entstehen können. Ein weiterer Nachteil der aus der EP-A-0 066 028 bekannten Vorrichtung ist außerdem, daß die in der Einrichtung vorhandenen Metallplatten wie Federn nur in einer Richtung arbeiten, indem sie die Beine in dem Hüft- und dem Kniegelenk strecken. Aus diesem Grund kann die genannte Vorrichtung nur dazu verwendet werden, den Patienten, die sich nicht selbständig bewegen können, Hilfe zu leisten. Das Einsatzgebiet der aus der EP-A-0 066 028 bekannten Vorrichtung ist also äußerst beschränkt.

Eine Vorrichtung zur Erleichterung des Gehens ist aus der SU-A-1 556 675 bekannt. Diese Vorrichtung weist eine Fixiereinrichtung auf, die in Form eines Korsetts mit an dessen Vorder- und Rückseite befindlichen Führungen ausgebildet ist. Auf den Führungen sind Buchsen verschiebbar und fixierbar angeordnet. Die konstruktive Ausführung der aus der SU-A-1 556 675 bekannten Vorrichtung sieht eine Fixierung des unteren Rumpfteils und der Beine vor. Daher ist diese Vorrichtung vortelihaft nur für die Bewegung von Schwerkranken einsetzbar. Außerdem besteht das ganze System aus steifen Materialien und wird in Kombination mit einem Korsett verwendet. Das macht die Vorrichtung sperrig und bietet keine Möglichkeit, die Bewegungsaktivität des Kranken beim Gehen zu vergrößern, weshalb der Einsatz der aus der SU-A-1 556 675 bekannten Vorrichtung für Behandlungszwecke problematisch ist.

Der Erfindung liegt die Aufgabe zugrunde, eine Vorrichtung zur Behandlung von Patienten mit Problemen der Haltung sowie der motorischen Aktivität zu schaffen, bei der die Fixierungselemente zur Verbindung der Halter eine Konstruktion haben, die es gestattet, die Fixierung des Rumpfes und der Extremitäten des Körpers des Patienten in einer Stellung zu halten, weiche den physiologischen Parametern nahekommt, wobei dem Patienten die Möglichkeit erhalten bleibt, energetisch belastete Bewegungen auszuführen, die für den betreffenden Patienten charakteristisch sind. Es wird dabei die Schaffung eines physiologisch normalen Stereotyps für Haltung und Bewegung bezweckt.

Erfindungsgemäß wird diese Aufgabe gelöst durch eine Vorrichtung nach Anspruch 1.

Die erfindungsgemäße Vorrichtung ermöglicht eine Fixierung der Gelenke in der erforderlichen Stellung, wobei ein Kraftmoment entsteht, das die Beugung, Streckung, Rotation sowie Adduktion und Abduktion der Extremitäten und des Rumpfes begünstigt.

Die erfindungsgemäße Vorrichtung kann z.B. zur Behandlung von Patienten mit zerebraler Kinderlähmung sowie bei Insulten mit Bewegungsstörungen verwendet werden.

Die erfindungsgemäße Vorrichtung erlaubt, die Fixierung praktisch aller Gelenke des Rumpfes und der Extremitäten in einer vorgegebenen Stellung zu halten, wobei gewährleistet wird, daß der Patient energetisch belastete Bewegungen mit für ihn möglichen Amplituden ausführen kann.

Die Vorrichtung weist Spannungsregler (Regelungsmittel) der elastischen Zugelemente auf, von denen jeder zwischen dem entsprechenden Zugelement und einem der Halter angeordnet ist.

Die Spannungsregler bieten die Möglichkeit, die Kraft der Einwirkung der Zugelemente auf das Knochen- und Muskelsystem zu verändern und individuell auszuwählen, wodurch sich die Behandlungseffektivität erhöht.

Die Verwendung der Vorrichtung gestattet es, die statischen (passiven) Korrektionen der pathologischen Stellungen von Rumpf und Extremitäten durch eine funktionelle (aktive) Korrektion zu ersetzen, gestaltet den früheren pathologischen Stereotyp der Haltung und der Bewegungen im Zentralnervensystem und an der Peripherie um, potenziert die Zerstörung eines alten, im Verlauf der Krankheit gebildeten pathologischen Blocks von Reflexen und die Schaffung von neuen Steuer- und Erregungsleitungssystemen vermittels der Erhaltungsstrukturen des Hirns. Außerdem besteht die Einwirkung der Vorrichtung auf den Organismus des Patienten darin, daß die Korrektion des Stütz- und Bewegungsapparates und die energetische Belastung der Bewegungen in der neuen Stellung der Extremitäten und des Rumpfes zur Aktivierung der zentralen Hirnstrukturen bei der Herausbildung einer neuen Organisation des Steuersystems für die Bewegungen sowohl des Stütz- und Bewegungsapparates als auch des motorischen Apparates des Sprachbildungssystems führt. Die Benutzung der erfindungsgemäßen Vorrichtung ermöglicht es, einen Stereotyp der Haltung und der Bewegungen zu schaffen, welcher dem physiologischen nahekommt.

Bei einer besonders bevorzugten Ausführungsform der erfindungsgemäßen Vorrichtung ist jedes Regelungsmittel der Spannung der elastischen Zugelemente in Form eines Bandes ausgebildet, dessen eines Ende mit dem elastischen Zugelement verbunden ist, während das andere Ende durch ein Schloß befestigt ist, das jeweils an einem der Halter angeordnet ist.

Weitere ziele und Vorteile der vorliegenden Erfindung ergeben sich aus der nachfolgenden ausführlichen Beschreibung eines Ausführungsbeispiels derselben und aus den beigefügten Zeichnungen. Es zeigt:

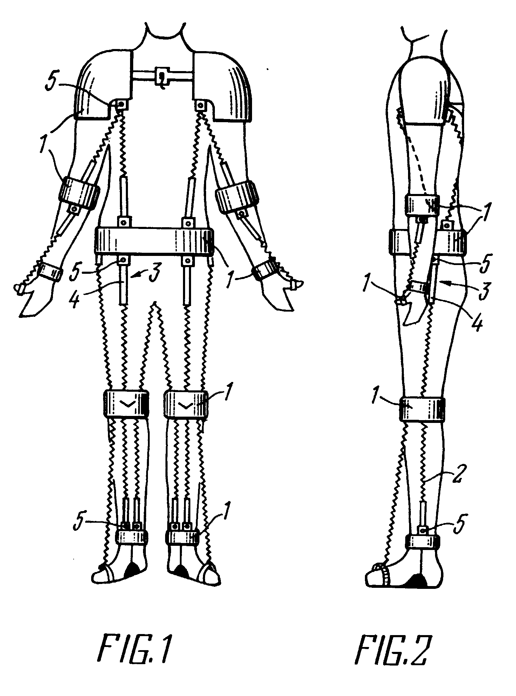

- Fig. 1:: die Vorderansicht der erfindungsgemäßen Vorrichtung;

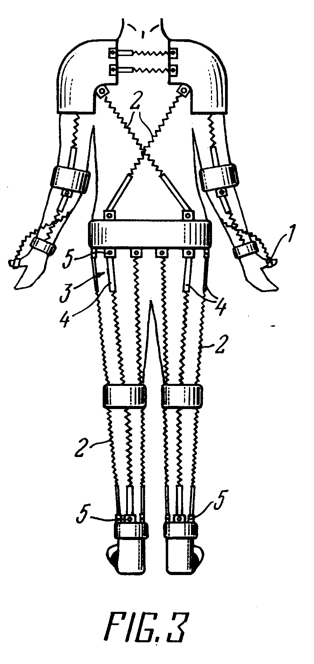

- Fig. 2:: die Seitenansicht;
- Fig. 3:: die Rückenansicht.

Die Vorrichtung umfaßt Halter 1, die zur Anbringung im Bereich der Oberarme, Ellbogen, Hände, der Lenden, Knie, Füße und Finger des Patienten bestimmt sind. Die Halter 1 sind mit Hilfe von Fixierungselementen untereinander verbunden, die als elastische Zugelemente 2 ausgebildet sind, weiche sowohl benachbarte als auch nichtbenachbarte Halter 1 verbinden können. Die Zugelemente 2 sind mit den Haltern 1 so verbunden, daß sie auf der Oberfläche des Körpers des Patienten in antagonistischen Paaren nach dem Prinzip der anatomischen Lage der Skelettmuskulatur angeordnet sind. Jedes Zugelement 2 umfaßt einen Regler (ein Regelungsmittel) 3 für seine Spannung, welcher dieses Zugelement 2 mit einem der Halter 1 verbindet.

Jeder Regler 3 ist in Form eines Bandes 4 ausgeführt, dessen eines Ende mit dem Zugelement 2 in Verbindung steht, während das andere in einem Schloß 5 befestigt ist, das wiederum in einem der Halter 1 befestigt ist. Der Regler 3 kann auch eine beliebige andere Konstruktion haben, die eine ähnliche Funktion erfüllt.

Die Halter 1 können aus einem beliebigen Material mit minimalem Spannungsgrad, beispielsweise aus Gewebe, Leder, Plast usw. bestehen.

Die Zugelemente 2 können aus Gummi oder Kunststoff bestehen, sie können aber auch in Form von metallischen Federn ausgebildet sein.

Die erfindungsgemäße Vorrichtung wird folgendermaßen benutzt:

Für einen Patienten wählt man individuell unter Berücksichtigung seines Zustands eine Vorrichtung entsprechender Größe, legt sie dem Patienten an und nimmt eine Spannung derjenigen Zugelemente 2 vor, weiche die Stellung von zu behandelnden Körperteilen korrigieren. Die Zugelemente 2 spannt man mit Hilfe der Regler 3 an und fixiert den erreichten Spannungszustand durch die Schlösser 5. Die Regelung der Zugelemente 2 nimmt man vor, bis eine neue Stellung des Rumpfes und der Extremitäten hergestellt ist, die der physiologischen nahekommt und bei der die Möglichkeit erhalten bleibt, Bewegungen mit einer Amplitude zu vollführen, welche sich der maximalen Amplitude bei diesem Patienten nähert. Die Spannkraft der Zugelemente 2 vergrößert man mindestens so lange, bis eine Belastung in der der jeweiligen Bewegung entsprechenden Muskelgruppe auftritt. Hiernach ist die Vorrichtung einsatzbereit.

Auf diese Weise erzeugt man mit Hilfe der Vorrichtung eine Stütz- und dynamische Struktur (funktionelles Korsett) und bereitet den Patienten auf die Ausführung von Bewegungen vor.

Die Vorrichtung wird von dem Patienten unter Berücksichtigung seines Zustands und seiner individuellen Besonderheiten täglich bis zu 12 Stunden pro Tag benutzt. Der Behandlungszyklus dauert 15 bis 30 Tage.

Die Zugelemente 2, die auf der Oberfläche des Körpers des Patienten in antagonistischen Paaren nach dem Prinzip der anatomischen verteilung der Skelettmuskulatur in bezug auf die Gelenke angeordnet sind, gewährleisten während der Benutzung der Vorrichtung alle Arten der Bewegung des Patienten. Im Laufe der Behandlung nimmt man eine dosierte Erhöhung des Spannungsgrades der Zugelemente 2 vor. Je nach Adaptation des Patienten an die korrigierende Einwirkung vergrößert man die Kraft der Korrektion so weit, daß sich der Patient auch bei Belastung noch wohl fühlt.

Während des Behandlungsprozesses bildet sich ein neuer Stereotyp der Bewegungssteuerung heraus. Außerdem wird die physiologische Stellung dominierend, was zu einer Senkung der pathologischen Muskelsynergien sowie einer Steigerung der motorischen Aktivität führt und die Möglichkeit bietet, die Haltung in den Fällen zu korrigieren, die sich durch andere Arten der Korrektion nicht verbessern lassen.

Die Vorrichtung kann durch einen Overall ergänzt werden, den der Patient über der Vorrichtung trägt, wobei im Overall Öffnungen für den Zugang zu den Reglern 3 vorgesehen sind.

### Beispiel 1:

Patient B.K., 17 Jahre alt. Diagnose: zerebrale Kinderlähmung, gestellt im Alter von sechs Monaten. Zum Zeitpunkt der Benutzung der erfindungsgemäßen Vorrichtung war beim Patienten eine Lähmung in Form von diplegia spastica herausgebildet. Es war eine dreifache Beugung in den unteren Extremitäten zu verzeichnen, verkompliziert durch Kontrakturen in den oberen Sprunggelenken, durch innere Verdrehung der Obersohenkel, nichtkompensierte Neigung des Körpers nach vorn, Fortbewegungsschwierigkeiten, pathologischen Gang, Equino-Valgus-Deformität beider Füße ("Schaukelfuß"), innere Verdrehung beider Arme, gestörte Motorik der Hände und Finger. Erhalten gebliebener Intellekt, satzweise Sprache. Der Patient war psychisch hochmotiviert für die Rehabilitation. Früher war eine medikamentöse Behandlung und eine physiotherapeutische Behandlung sowie eine Korrektion durch Gipsbinden und Longuetten durchgeführt worden. Diese durchgeführten Behandlungen erzielten einen vorübergehenden Effekt.

Der Patient machte eine Kur mit der erfindungsgemäßen Vorrichtung während eines Monats, täglich 2 bis 3 Stunden pro Tag.

Die erzeugte Belastung wurde vom Patienten während der ersten sieben Tage empfunden, worauf die Empfindung der Belastung verschwand, es trat eine Adaptation ein. Allerdings kam in den ersten fünf Tagen die pathologische Einstellung des Rumpfs und der Extremitäten zwei Stunden nach der Wegnahme der Belastung erneut zum Vorschein. Am zehnten Tag der Anwendung der Behandlung zeigte sich ein stabiles Resultat in Form einer vollständigen Beseitigung der pathologischen Körperhaltung, eine Überwindung der gebeugten Haltung der unteren Extremitäten, Verbesserung des Gangablaufs, erleichtetes Vorwärtsbewegen der Oberschenkel, erhöhte Geschwindigkeit beim Gehen. Nach dem zehnten Tag begann der Patient in aufrechter vertikaler Haltung zu gehen. Außerdem ging beim Patienten ab dem zehnten Tag der Behandlung die Pronationsstellung der Arme zurück, und die Bewegungen der Hände und Finger verbesserten sich. Der Patient wurde nach dreißig Tagen mit bedeutend verbesserten Bewegungs- und statischen Funktionen nach Hause entlassen.

Nachstehend ist eine Auflistung der klinischen Effekte beim Einsatz der erfindungsgemäßen Vorrichtung für eine Gruppe von Patienten mit residualem Spätstadium der zerebralen Kinderlähmung angeführt. (Alter der Patienten 15 bis 20 Jahre, Zahl der Behandlungssitzungen 16 bis 22 im Laufe eines Monats).

### 1. Erkrankungsform: Diplegia spastica

### Anzahl der Patienten: 4

### Dynamik im Prozeß der Regenrationskur:

1. Normalisierung (nicht immer vollständig) der pathologischen Hauptmuskelsynergien, die eine dreifache Beugung der Beine und eine Beugesynergie im Arm bedingen. Vergrößerung der Schrittlänge, richtige Formierung des vorderen und hinteren Abstoßes des Schritts.
2. Abnahme der Torsion des Schultergürtels und des Rumpfes. Die Intensität des frontalen Schaukelns geht zurück.
3. Verschwinden der pathologischen Synergie im Schultergürtel. Vergrößerung der Manipulationsmöglichkeiten der Hand und der Finger.
4. Allmähliche Verbesserung der Aussprache beim Reden.

¹ Bei diesem Wertungssystem ist 5 die höchste erreichbare Punktzahl.

### Bewertung des Effektes nach dem Fünfpunkte-Wertungssystem¹: 4 - 3

### 2. Erkrankungsform: Hyperkinetische Form

### Anzahl der Patienten: 2

### Dynamik im Prozess der Regenerationskur:

1. Beginn des Gehens ohne Unterstützung. Torsions-Hyperkinesen der Hals- und Rumpfmuskeln bleiben noch erhalten.
2. Nach 10 bis 15 Behandlungssitzungen nimmt die Intensität der Hyperkinesen ab. Gehen ist möglich, auch über größere Entfernungen, aber ohne begleitende Bewegungen der Arme.
3. Verbesserung der Sprache (Sprache wird für mit dem Patienten bekannte Menschen verständlich). Es beginnen Armbewegungen, mit deren Hilfe der Patient alltägliche Verrichtungen durchführen kann.

### Bewertung des Effektes nach dem Fünfpunkte-Wertungssystem: 4-3

### 3. Erkrankungsform: Zerebellare Form

### Anzahl der Patienten: 2

### Dynamik im Prozeß der Regenerationskur:

1. Selbständiges Gehen über Entfernungen von 200 bis 300 m (Anfangszustand: Fehlen des Gehens)
2. Abnahme des zerebellaren Symptombildes (Hypermetrie, Dysmetrie, Ataxis des Rumpfes und der Extremitäten).
3. Abnahme des Skandierens der Sprache. Sprache wird deutlich.

### Bewertung des Effekts nach dem Fünfpunkte-Wertungssystem: 5

### 4. Erkrankungsform: Hemiparetische Form

### Anzahl der Patienten: 2

### Dynamik im Prozeß der Regenerationskur:

1. Verbesserung der vorhandenen Bewegungsfunktionen.
2. Verminderung der Ermüdbarkeit beim Gehen, Gehen über größere Entfernungen.
3. Verbesserung der Manipulationsmöglichkeit des paretischen Arms.

### Bewertung des Effektes nach dem FünfpunkteWertungssystem: 3

Wie aus der Tabelle erkennbar, sind die besten Erfolge der Behandlung mit der erfindungsgemäßen Vorrichtung bei der zerebellaren Erkrankungsform erzielt worden, der geringste Effekt ist bei der hemiparetischen Form der zerebralen Kinderlähmung registriert worden.

### Beispiel 2

Patient X, 40 Jahre alt. Diagnose: Lendenmarkaffektion mit teilweiser Störung der Leitung (Caissonkrankheit).

Der Patient machte eine Kur unter Benutzung der erfindungsgemäßen Vorrichtung. Die Behandlung führte zur Verbesserung der Gangparameter: Auftreten einer Synchronisation der Muskelarbeit, Erhöhung der Gehgeschwindigkeit, verringerte Ermüdbarkeit.

## Patentansprüche

1. Vorrichtung zur Behandlung von Patienten mit einer Störung der Körperhaltung und der motorischen Aktivität, mit für das Becken und die Füße bestimmten Haltern (1), die als elastische Zugelemente (2) ausgebildete Fixierungselemente aufweisen, dadurch gekennzeichnet, daß
- die Vorrichtung in Form eines funktionellen Stütz- und Bewegungskorsetts ausgebildet ist und zusätzliche Halter (1) für Schultern, Knie, Ellenbogen, Hände und Finger aufweist, wobei alle Halter (1) mit Hilfe der elastischen Zugelemente (2) untereinander verbunden sind, und
- an jedem elastischen Zugelement (2) ein Regelungsmittel (3) für die Spannung befestigt ist, welches das elastische Zugelement (2) mit einem der Halter (1) verbindet, wobei die elastischen Zugelemente (2), die mit den Haltern (1) verbunden sind, an der Körperoberfläche des Patienten in antagonistischen Paaren relativ zu Gelenken nach dem Prinzip der anatomischen Lage der Skelettmuskulatur anzuorden sind.

2. Vorrichtung nach Anspruch 1, dadurch gekennzeichnet, daß jedes Regelungsmittel (3) der Spannung der elastischen Zugelemente in Form eines Bandes (4) ausgebildet ist, dessen eines Ende mit dem elastischen Zugelement (2) verbunden ist, während das andere Ende durch ein Schloß (5) befestigt ist, das jeweils an einem der Halter (1) angeordnet ist.

## Claims

1. An apparatus for treating patients with a disorder affecting posture and motor activity, with retainers (1) which are intended for the pelvis and the feet and which have securing elements in the form of elastic tension elements (2), characterised in that
- the apparatus is in the form of a functional brace and movement corset and has additional retainers (1) for the shoulders, knees, elbows, hands and fingers, wherein all the retainers (1) are connected to one another by means of the elastic tension elements (2), and
- a regulating means (3) for the tension is fastened to each elastic tension element (2), which regulating means connects the elastic tension element (2) to one of the retainers (1), wherein the elastic tension elements (2), which are connected to the retainers (1), are to be disposed on the patient's body surface in counteracting pairs relative to joints in accordance with the principle of the anatomical position of the skeletal muscle.

2. An apparatus according to Claim 1, characterised in that each regulating means (3) for the tension of the elastic tension elements (2) is in the form of a strip (4), one end of which is connected to the elastic tension element (2), whereas the other end is fastened by a buckle (5) which in each case is provided on one of the retainers (1).

## Revendications

1. Dispositif pour le traitement de patients présentant une perturbation de l'attitude corporelle et de l'activité motrice, comportant des supports (1) définis pour le bassin et les pieds, qui présentent des éléments de fixation configurés comme éléments élastiques de traction (2), caractérisé en ce que
- le dispositif est configuré sous forme d'un corset fonctionnel de soutien et de déplacement et présente des supports supplémentaires (1) pour les épaules, les genoux, les coudes, les mains et les doigts, tous les supports (1) étant reliés les uns avec les autres à l'aide des éléments élastiques de traction (2), et
- à chaque élément élastique de traction (2) est fixé un moyen de réglage (3) de a tension, qui relie l'élément élastique de traction (2) à l'un des supports (1), les éléments élastiques de traction (2) qui sont reliés au support (1) devant être disposés à la surface du corps du patient en paires antagonistes par rapport à des articulations, suivant le principe de la position anatomique de la musculature du squelette.

2. Dispositif selon la revendication 1, caractérisé en ce que chaque moyen de réglage (3) de a tension des éléments de traction élastiques est configuré en forme d'une bande (4) dont une extrémité est reliée à l'élément élastique de traction (2), tandis que l'autre extrémité est fixée par un fermoir (5) qui est chaque fois disposé sur l'un des supports (1).
